# EUROPEAN PATENT APPLICATION

(11) **EP 4 177 906 A1**
(43) Date of publication of application: **10.05.2023**
(21) Application number: 21306542.8
(22) Date of filing: 03.11.2021
(51) Int. Cl.: G16H 50/20, G16H 50/30

(54) **METHOD, COMPUTER PROGRAM, DEVICE AND SYSTEM FOR PREDICTING ASTHMA EXACERBATION**

(71) Applicant: Bull SAS, 78340 Les Clayes-sous-Bois (FR)
(72) Inventor: JIMENEZ, Natalia, CAMBRIDGE (GB)
(74) Representative: IPAZ

(57) **Abstract**

The invention relates to a computer implemented method (100) for predicting asthma exacerbation for a person having asthma, said method (100) comprising at least one iteration of a prediction phase (102) comprising the following steps:
- receiving (110) at least one input data, called respiratory data, relating to a breathing capacity of said person,
- receiving (108) at least one input data, called weather data, relating to a weather condition; and
-computing (112), by a predetermined prediction model taking said input data, of a risk data relating to an occurrence of asthma exacerbation for said person.

The invention further relates to a computer program, a device and a system implementing such a method.

## Description

### Field of the invention

The present invention relates to a computer implemented method for predicting asthma exacerbation for a person having asthma. The invention further relates to a computer program, a device and a system implementing such a method.

The field of the invention is the field of digital solutions for predicting risk of asthma exacerbation for a person.

### Background

Asthma is a common chronic respiratory disease affecting 1-18% of the population of different countries. Asthma symptoms are caused by an inflammation and narrowing of the small airways in the lungs and comprise a combination of cough, wheeze, shortness of breath and chest tightness. An estimated 262 million people suffered from asthma in 2019 and caused 461,000 deaths. This figure is set to rise to 400 million asthma sufferers in 2025. Asthma exacerbations remain a major reason for health care utilization and a significant burden to patients and society. Therefore, there is a pressing need to develop strategies to prevent these events.

Actors in this field have already proposed action plans or digital solutions such as smart inhalers, or wearables, that sense breathing difficulties to monitor the symptoms and ensure medication.

However, these solutions have limited performance and do not help persons suffering from asthma before an exacerbation occurs.

A purpose of the present invention is to overcome at least one of these drawbacks.

Another purpose of the invention is to provide a solution for predicting asthma exacerbation before said exacerbation occurs.

Another purpose of the invention is to provide a more efficient solution for predicting asthma exacerbation before said exacerbation occurs.

### Summary of the invention

The invention makes it possible to achieve at least one of these aims by a computer implemented method for predicting asthma exacerbation for a person having asthma, said method comprising at least one iteration of a prediction phase comprising the following steps:
- receiving at least one input data, called respiratory data, relating to a breathing capacity of said person,
- receiving at least one input data, called weather data, relating to a weather condition; and
- computing, by a predetermined prediction model, as a function of said input data, of a risk data relating to an occurrence of asthma exacerbation for said person.

The present invention makes it possible to predict risk of occurrence of an asthma exacerbation for a person having asthma according to at least respiratory concerning the person and weather data. Thus, thanks to the present invention, the person having asthma may have indication regarding the occurrence of a potential exacerbation, before said exacerbation occurs.

Plus, the present invention proposes to compute exacerbation occurrence risk data according to input data regarding several parameters, comprising at least personal respiratory data and weather data. Thus the risk of occurrence is predicted in a more efficient and more precise manner.

Moreover, the risk data is computed by a predetermined model that is previously trained on asthma exacerbation prediction by using data related to previous exacerbations happened in the past, for the same person or for different persons. Thus, the use of such a predetermined model makes it possible to more efficiently predict the occurrence of asthma exacerbations.

According to non-limitative embodiments, the risk data may be a probability data regarding the occurrence of an asthma exacerbation. For example, the probability data may be a percentage comprised between 0% ad 100%.

In other non-limitative embodiments, the risk data may be a classification data relating to at least two classes: "risky" and "not risky". Of course, there may be more than two classes regarding the risk of exacerbation such as: "very low", "low", "medium", "high" and "very high".

Other type of risk data may be used without departing from the scope of the present invention.

According to an advantageous embodiment, the prediction phase may be implemented for the current moment, or for a moment in the future.

For example, a person having asthma may want to know the risk of occurrence of an exacerbation according to current weather conditions.

Alternatively, the person having asthma may want to know the risk of occurrence of an exacerbation for a time in the future, for example for the next day or the next week, according to the forecasted weather conditions.

According to an advantageous embodiment, the prediction phase may also comprise a step of receiving an input data, called location data, relating to a geographical location, said weather data relating to a weather condition in said geographical location.

Thus, the computation of the risk data is more precise by taking into account a more precise geographical location, as more precise weather data may be gathered knowing the geographical location of the person.

The geographical location may be the current location of the person or a future location of the person. For example, if the person plans to go to a specific geographical location, he/she may first determine the risk of occurrence of exacerbation before going to said geographical location.

According to a non-limitative example, the person may want to visit Paris the next day. He/she may first compute the risk of occurrence of an exacerbation according to:
- his/her respiratory data,
- weather conditions forecasted for Paris the next day, and
- optionally the current weather conditions.
Thus, the pre-trained prediction model may take all of these data and output a risk data regarding the occurrence of an exacerbation for him/her in Paris, next day. If the risk is too high, the person may renounce to going to Paris the next day.

The location data may be entered manually by the person.

Alternatively, the location data may be collected from a module, such as a GPS module, or a software application, dedicated or not to the present invention. For example, the GPS module, respectively the software application, may be integrated in a device carrying out the method according to the invention, or in a user device in communication with the device carrying out the method according to the invention.

Such a device may be a user terminal, such as a Smartphone, a tablet, a Smartwatch, etc. Alternatively, such a device may be a wearable apparatus dedicated to carry out the method according to the invention, such an electronic wristband, an electronic necklace, etc.

According to advantageous non-limitative embodiments, the method according to the invention may also comprise a step of receiving input data, called clinical data, relating to a clinical past of the person, the prediction model also taking into account said clinical data for computing the risk data.

Such clinical data may be indicative of the type of asthma, or the severity level of asthma, the person has.

Such clinical data may be indicative of a sensibility of a person to a given parameter leading to asthma exacerbation. For example, clinical data may indicate that the person has a specific sensibility to pollen such that even a low level of pollen may yield to exacerbation. For example, clinical data may indicate a value of pollen count that may lead to asthma exacerbation for the person.

Clinical data may be specific data related to asthma.

Alternatively, clinical data may be raw data regarding clinical past of the person. In this case, the method according to the invention may also comprise, before the first iteration of the prediction phase, a step of processing the clinical data for identifying data related to asthma the person has, such as data regarding the type or the severity of the asthma of the person, a parameter identified as triggering an exacerbation, etc.

The clinical data may be processed manually, for example by the person himself or by an operator or a medical person.

Alternatively, the method according to invention may comprise a natural language processing, NLP, of the clinical data.

Such a natural language processing may be carried out by known techniques such as statistical NLP, machine learning, and deep learning. For example, the NLP may be carried out by using word segmentation or tokenization, lexical or relational semantics, etc.

At least one clinical data may be entered manually.

At least one clinical data may be collected, or received, from a local or distant database, through a communication network.

According to non-limitative embodiments, the weather data may comprise data relating to:
- pollutants and/or pollen present in the air, and/or
- climate data, especially a temperature data and/or a humidity data.

For example, weather data may comprise:
- a value regarding the quantity of pollen,
- a value regarding the amount of a pollutant,
- a humidity level,
- etc.

At least one weather data may be entered manually.

At least one weather data may be collected, or received, from local or distant server, or a local or distant weather application or weather station, through a communication link, or a communication network such as the Internet.

Alternatively, the weather data may be collected from a module, such as a weather module or a weather application, dedicated or not to the present invention. For example, the weather module, respectively the weather application, may be integrated in a device carrying out the method according to the invention, a local user device in communication with the device carrying out the method according to the invention.

Such a device may be a user terminal, such as a Smartphone, a tablet, a Smartwatch, etc. Alternatively, such a device may be a wearable apparatus dedicated to carry out the method according to the invention, such an electronic wristband, an electronic necklace, etc.

The respiratory data may be, or comprise:
- the amount (volume), and/or
- the speed (flow);
of air that can be inhaled and exhaled by the person.

The respiratory data may be collected from a spirometer, or similar.

The prediction model may be a pre-trained neural network.

The neural network may be a Convolutional Neural Networks (CNN) used to classify the different combination of input data.

The neural network may for example comprise about 50 layers.

The input data may be entered to the neural network as a data vector, noted IV (for Input Vector), comprising
- at least one respiratory data, noted R, concerning the person;
- at least one weather data, noted W; and
- optionally, at least one clinical data concerning the person, noted C.
such as the input data vector IV may be noted: IV= {R;W;C}.

The neural network may be trained using a training database TDB, comprising several training data sets, noted TDS₁-TDSₘ. Each training data set TDSᵢ may comprise:
- a training input data vector TIVᵢ comprising the same type of data as indicated above for the input vector IV; and
- an associated data relating to the risk of exacerbation.

At least one training data set TDS may be obtained during a previous asthma exacerbation of the same person or a different person. Such training data sets may be collected from different asthma patients from all over the world. Such TDS may be collected from previous clinical investigations on asthma and asthma patients.

At least one training data set TDS may be obtained by simulation, or defined by doctors or researchers, etc.

The neural network may be trained using the backpropagation algorithm with a predetermined cost function (or loss function), which may the sum of the errors for each TDS.

The error for a training data set TDSᵢ may be a distance, for exempla a cosine distance, between the risk data of said TDSᵢ and the output risk data for the training input data vector TIVᵢ of this TDSᵢ.

The method according to the invention may comprise a training phase comprising several iterations of a training step. Each training step may comprise the following operations:
- test of the neural network on each training data set TDSᵢ;
- computation of the value of the cost function as a function of the calculated error for each training data set TDSᵢ;
- changing the value of the weights of the neural network.

The training phase may be terminated if the cost function doesn't decrease for at least five (5) consecutive training steps.

Alternatively, the training may be carried out by using a first part, called learning part, of the training database TDB. The remaining part, called test part, of the training database TDB may be used to validate, or not, the training of the neural network. In this case, an accuracy threshold may be defined in order to consider the neural network trained enough. For example, the accuracy level may be at least 75%, preferably 80%, and more preferable 85% of accuracy.

Alternatively, the prediction model may be a classification tree used to classify the input data in at least two classes.

According to yet another alternative, the prediction model may be a table storing different values of risk in connection with different combinations of input data.

According to another aspect of the same invention, it is proposed a computer program comprising instructions, which when executed by a computer, cause the computer to carry out the steps of the method according to the invention.

The computer program may be in any programming language such as C, C++, JAVA, Python, etc.

The computer program may be in machine language.

The computer program may be stored, in a non-transient memory, such as a USB stick, a flash memory, a hard-disc, a processor, a programmable electronic chop, etc.

The computer program may be stored in a computerized device such as a Smartphone, a tablet, a computer, a server, etc.

The computer program according to the invention may comprise one or several applications to carry out the method according to the invention. For example, the computer program may comprise:
- a first input application for collecting, or receiving, respiratory data, for example from a spirometer or a similar device,
- a second input application for collecting, or receiving, weather data from a weather device or weather application;
- a third input application for collecting, or receiving, location data from a location device or application, such as a GPS module;
- a fourth input application for collecting, or receiving, and optionally treating, clinical data from a server or a database storing such data;
- an application for executing the predetermined prediction model providing a risk data relating to an occurrence of asthma exacerbation for said person.
At least two input applications may be integrated in a single input application.

According to another aspect of the same invention, it is proposed a device for predicting asthma exacerbation for a person having asthma comprising means configured to carry out the steps of the method according to the invention.

The device may be a personal device such as a Smartphone, a tablet, a Smartwatch, a computer, any wearable electronic device, etc.

The device according to the invention may execute one or several applications to carry out the method according to the invention.

The device according to the invention may be loaded with, and configured to execute, the computer program according to the invention.

According to another aspect of the same invention, it is proposed a system for predicting asthma exacerbation for a person having asthma, said system comprising:
- at least one respiratory data sensor, such as a spirometer;
- at least one means for providing weather data, such as weather application; and
- a device according to the present invention for providing an asthma exacerbation risk data as a function of input data provided by said means.

The system according to the invention may comprise any combination of at least one of the futures described above in connection with the invention, that are not repeated here for sake of brevity.

For example, in brief, the system may comprise a combination of at least one of the following features:
- a localization module, such as a GPS module, providing location data;
- a clinical data module, or database, for providing clinical data;
- etc.
or any of the features described above in connection with these features.

### Description of the figures and embodiments

Other advantages and characteristics will become apparent on examination of the detailed description of an embodiment which is in no way limitative, and the attached figures, where:
- Figure 1 is a diagrammatic representation of a non-limitative example of a method according to the invention;
- Figure 2 is a diagrammatic representation of a non-limitative example of a device according to the present invention; and
- Figure 3 is a diagrammatic representation of a non-limitative example of a system according to the present invention.

It is well understood that the embodiments that will be described below are in no way limitative. In particular, it is possible to imagine variants of the invention comprising only a selection of the characteristics described hereinafter, in isolation from the other characteristics described, if this selection of characteristics is sufficient to confer a technical advantage or to differentiate the invention with respect to the state of the prior art. Such a selection comprises at least one, preferably functional, characteristic without structural details, or with only a part of the structural details if this part alone is sufficient to confer a technical advantage or to differentiate the invention with respect to the prior art.

In the FIGURES, elements common to several figures retain the same reference.

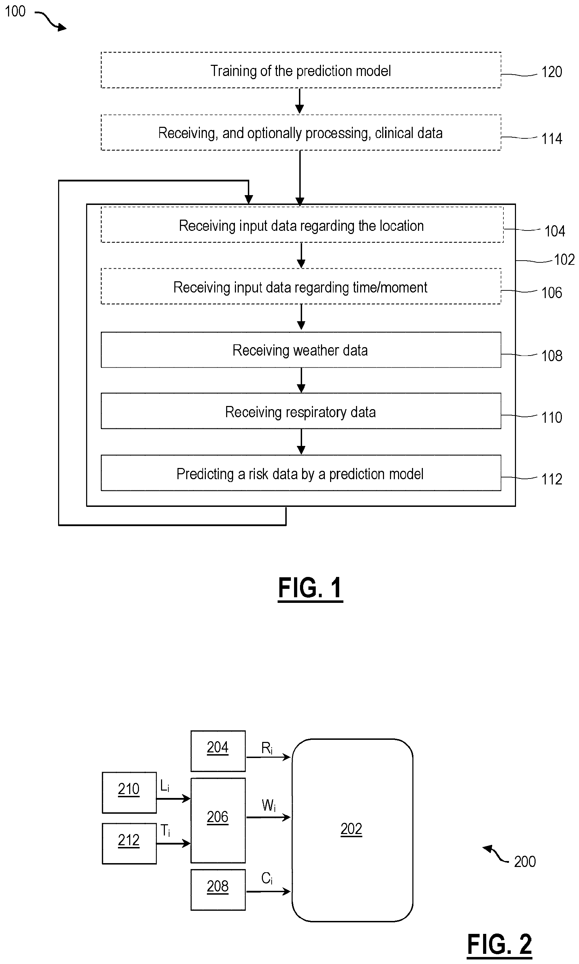

FIGURE 1 is a diagrammatic representation of a non-limitative example of a method according to the present invention.

The method 100, shown in FIGURE 1, may be used to predict the risk of occurrence of asthma exacerbation for at least one person with asthma disease.

The method comprises at least one iteration of a prediction phase 102. The prediction phase 102 may be executed as many times as wanted in order to determine asthma exacerbation risk for the person.

The prediction phase 102 is realised for a given location.

The prediction phase 102 may comprise an optional step 104 for receiving input data regarding the location concerned by the prediction.

The location for the prediction may be the current location, or another location for example, the location may be another town where the person wants to go in the future, for example the next day or the next week.

When the location is the current location, the input location data may be provided by a GPS module of the device implementing the method 100, or of another device, such as for example a user device such as a Smartphone, a tablet, a Smartwatch, etc.

When the location is not the current location, the input location data may be provided by a digital agenda, or another application.

Alternatively, regardless the location, the location data may be entered manually by the user, for example through a user interface.

The prediction phase 102 is realised for a given time/moment.

The prediction phase 102 may comprise an optional step 106 for receiving input data regarding the moment concerned by the prediction.

The moment for the prediction may be the current time, or another time in the future, for example the next day or the next week.

When the prediction moment is the current moment, the data regarding the prediction time may be provided by a time clock of the device implementing the method 100, or of another device such as for example a user device such as a Smartphone, a tablet, a Smartwatch, etc.

When the prediction moment is not the current moment, the input time data may be provided by a digital agenda, or another application. For example, the time may be the next day, or the next week.

Regardless the moment concerned by the prediction, the prediction moment data may be entered manually by the user, for example through a user interface.

The prediction phase 102 comprises a step 108 for receiving input data, called weather data, relating to a weather condition, in the location and the time indicated in steps 104 and 106. If the steps 104 and 106 are not realized, then the weather condition relates to current time and the current location.

The weather data may comprise data relating to:
- a value regarding the quantity of pollen,
- a value regarding the amount of a pollutant,
- a humidity level,
- a temperature,
- etc.

At least one weather data may be entered manually.

At least one weather data may be collected, or received, from local or distant server, or a local or distant weather application or weather station, through a communication link, or a communication network such as the Internet.

Alternatively, the weather data may be collected from a module, such as a weather module, or a weather application, dedicated or not to the present invention. For example, the weather module, respectively the weather application, may be integrated in a device carrying out the method according to the invention, a local user device in communication with the device carrying out the method according to the invention.

Such a device may be a user terminal, such as a Smartphone, a tablet, a Smartwatch, etc. Alternatively, such a device may be a wearable apparatus dedicated to carry out the method according to the invention, such an electronic wristband, an electronic necklace, etc.

The prediction phase 102 comprises a step 110 for receiving input data, called respiratory data, concerning the breathing capacity of the person. The respiratory data may comprise the amount (volume) and/or the speed (flow) of air that can be inhaled and/or exhaled by the person. The respiratory data may be provided by a spirometer, or another apparatus, carried or worn by the person.

The prediction phase 102 comprises a step 112 for predicting, with a pre-determined prediction model and as a function of input data, a risk of asthma exacerbation for the person. The risk data may be of any kind. For example, the risk data may be a probability percentage, the value of which is comprised between 0% and 100%. Alternatively, and more preferably, the risk data may be a classification data among at least two classes, such as "very low", "low", "medium", "high" and "very high".

The method 100 may comprise, before the first iteration of the prediction phase 102, a step 114, for receiving clinical data concerning the person, such as the type of asthma, or the severity level of asthma, the person has. In this case, the prediction model provides the risk data as a function of input clinical data related to asthma the person has.

When the received data is raw data regarding clinical past of the person, said step 114 may comprise processing the clinical data in order to extract data related to the asthma the person has. The processing of the clinical data may be carried out manually. Alternatively, the processing of the clinical data may be done automatically, for example by natural language processing, NLP.

The prediction model used in step 112 may be a pre-trained neural network.

For example, the prediction model may be a convolutional Neural Networks (CNN) used to classify the different combinations of input data in order to provide a classification data, as indicated above.

The neural network may for example comprise about 50 layers.

The input data may be entered to the neural network as a data vector, noted IV. Each input vector IVᵢ may comprise:
- at least one respiratory data, noted Rᵢ, concerning the person;
- at least one weather data, noted Wᵢ, for a given location and a given time;
- optionally, at least one clinical data concerning the person, noted Cᵢ.
such as IVᵢ= {Rᵢ;Wᵢ;Cᵢ}.

The neural network may provide a risk data, noted RD. For example, the risk data RDᵢ may take several values such as: "very low", "low, "medium", "high" and "very high".

The neural network may be trained using a training database, noted TDB, comprising several training data sets, noted TDS. Each training data set TDSᵢ may comprise:
- a training input data vector TIVᵢ comprising the same type of data as those indicated above for the input vector IV; and
- an associated training risk data, noted TRDᵢ, relating to the risk of exacerbation;
such as TDSᵢ={TIVᵢ;TRDᵢ}, and TIVᵢ={TRᵢ;TWᵢ;TCᵢ}, with:
- TRᵢ, at least one training respiratory data of the training data set TDSᵢ;
- TWᵢ, at least one training weather data of the training data set TDSᵢ;
- TCᵢ, at least one optional clinical training data concerning the person, of the training data set TDSᵢ.

For example TRDᵢ may take several values, such as "very low", "low, "medium", "high" and "very high".

At least one training data set TDSᵢ may be obtained during a previous asthma exacerbation of the same person or a different person. Such training data sets may be collected from different asthma patients from all over the world. Such TDSᵢ may be collected from previous clinical investigations on asthma and asthma patients.

At least one training data set TDSᵢ may be obtained by simulation, or defined by doctors or researchers, etc.

The neural network may be trained using the backpropagation algorithm with a predetermined cost function (or loss function), which may the sum of the errors for each TDSᵢ.

The method 100 may comprise a training phase 120 comprising several iterations of a training step. Each training step may comprise the following operations:
- test of the neural network on each training data set TDSᵢ;
- computation of the value of the cost function as a function of the calculated error for each training data set;
- changing the value of the weights of the neural network.

The training phase 120 may be terminated if the cost function doesn't decrease for at least five (5) consecutive training steps.

FIGURE 2 is a diagrammatic representation of a non-limitative example of a device according to the present invention.

The device 200, shown in FIGURE 2, may be used to carry out a method for predicting asthma exacerbation for a person having asthma, and in particular the method 100 of FIGURE 1.

The device 200 may comprise a prediction module 202 executing the prediction model.

Moreover, the device 200 of FIGURE 2 may comprise a module 204 for receiving, or collecting, respiratory data, Rᵢ, for example from a spirometer or a similar device. In some embodiments, the device 200 may also comprise such a spirometer.

The device 200 may comprise a module 206 for receiving, or collecting, weather data, Wᵢ, from a weather device or weather application. In some embodiments, the device 200 may also comprise such a weather device or weather application.

The device 200 may optionally comprise a module 208 for receiving, or collecting, and optionally processing, clinical data Cᵢ from a server or a database storing such data. In some embodiments, the device 200 may also comprise such a server or a database.

Optionally, the device 200 may also comprise a module 210 for receiving, or collecting, location data, Lᵢ, for example from a GPS module. In some embodiments, the device 200 may also comprise such a GPS module. The location data Lᵢ may be used to receive or collect the weather data Wᵢ.

Optionally, the device 200 may also comprise a module 212 for receiving, or collecting, time data, Tᵢ, for example from a time clock. In some embodiments, the device 200 may also comprise such a time clock. The time data may be used to receive or collect the weather data Wᵢ.

At least one of the modules may be a software module, such as a computer program or an application.

At least one of the modules may be a hardware module, such as a processor, à chip, or a calculator.

At least one of the modules may be an individual module.

At least two modules may be integrated in a same module.

The device according to the invention may be a user device such as a Smartphone, a tablet, a smartwatch. The device according to the invention may be an apparatus, or a clothes, worn by the user such as a wristband or necklace.

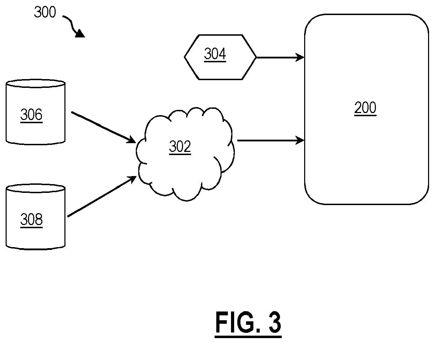

FIGURE 3 is a diagrammatic representation of a non-limitative example of a system according to the present invention.

The system 300, shown in FIGURE 3, may be used to carry out a method for predicting asthma exacerbation for a person having asthma, and in particular the method 100 of FIGURE 1.

The system 300 may comprise a device according to the invention, and in particular the device 200 of FIGURE 2.

Moreover, the system 300 may comprise a spirometer 304, or a similar device, or any other apparatus, for providing respiratory data to the device 200, as input data.

Optionally, but preferably, the system 300 may comprise at least one of the following components:
- a server 306 for providing weather data for a specific location and at a specific time or moment. The weather server 306 may be a local server connected to the device 200, through wired or wireless connexion. Alternatively, as represented in FIGURE 3, the weather server 306 may be a distant server in communication with the device 200 through a communication network, such as the Internet 302; and
- a server 308 comprising a database for providing clinical data for the person, especially clinical data related to the asthma the person has. The clinical data server 308 may be a local server connected to the device 200, through wired or wireless connexion. Alternatively, the clinical data server 308 may be a distant server in communication with the device 200 through a communication network, such as the Internet 302.

Of course, the invention is not limited to the examples detailed above.

## Claims

1. A computer implemented method (100) for predicting asthma exacerbation for a person having asthma, said method (100) comprising at least one iteration of a prediction phase (102) comprising the following steps:
- receiving (110) at least one input data, called respiratory data, relating to a breathing capacity of said person,
- receiving (108) at least one input data, called weather data, relating to a weather condition; and
- computing (112), by a predetermined prediction model, as a function of said input data, of a risk data relating to an occurrence of asthma exacerbation for said person.

2. The method (100) according to the preceding claim, **characterized in that** the prediction phase (102) also comprises a step (104) of receiving an input data (Lᵢ), called location data, relating to a geographical location, said weather data (Wᵢ) relating to a weather condition in said geographical location.

3. The method (100) according to any one of the preceding claims, **characterized in that** said method also comprises a step (114) of receiving input data (Cᵢ), called clinical data, relating to a clinical past of the person, the prediction model also taking into account said clinical data (Cᵢ) for computing the risk data.

4. The method (100) according to the preceding claim, **characterized in that** said method (100) also comprises, before the first iteration of the prediction phase, a step (114) of processing the clinical data for identifying data related to asthma the person has.

5. The method (100) according to the preceding claim, **characterized in that** the step (114) of processing the clinical data comprises natural language processing, NLP, of said clinical data.

6. The method (100) according to any one of the preceding claim, **characterized in that** the weather data (Wᵢ) comprises data relating to:
- pollutants and or pollen present in the air, and/or
- climate data, especially a temperature data and/or a humidity data.

7. The method (100) according to any one of the preceding claim, **characterized in that** the respiratory data (Rᵢ) is collected from a spirometer (304).

8. The method (100) according to any one of the preceding claims, **characterized in that** the prediction model is a pre-trained neural network.

9. Computer program comprising instructions, which when executed by a computer, cause the computer to carry out the steps of the method (100) of any one of the preceding claims.

10. Device (200) for predicting asthma exacerbation for a person having asthma comprising means configured to carry out the steps of the method (100) of any one of the preceding claims.

11. System (300) for predicting asthma exacerbation for a person having asthma, said system (300) comprising:
- at least one respiratory data sensor means (304);
- at least one means (306) for providing weather data, such as weather application; and
- a device (200) according to the preceding claim for providing an asthma exacerbation risk data as a function of input data provided by said means.
